## Europäisches Patentamt
## European Patent Office
## Office européen des brevets

(11) Numéro de publication: **0 152 338**
**B1**

(12) **FASCICULE DE BREVET EUROPÉEN**

(45) Date de publication du fascicule du brevet:
12.07.89

(21) Numéro de dépôt: **85400173.2**

(22) Date de dépôt: **01.02.85**

(51) Int. Cl.⁴: **A 61 K 6/06**

(54) **Fritte pour la fabrication d'une couche céramique superficielle transparente d'une reconstitution céramo-métallique dentaire.**

(30) Priorité: **15.02.84 FR 8402297**

(43) Date de publication de la demande:
**21.08.85 Bulletin 85/34**

(45) Mention de la délivrance du brevet:
**12.07.89 Bulletin 89/28**

(84) Etats contractants désignés:
**AT BE CH DE FR GB IT LI LU NL SE**

(56) Documents cité:
**US-A-4 159 358**

**CHEMICAL ABSTRACTS, vol. 93, no. 10, 8 septembre 1980, page 393, no. 101507w, Columbus, Ohio, US; & JP - A - 80 03301 (WADA SEIMITSU-SHIKEN CO., LTD.) 11-01-1980**
**DERWENT JAPANESE PATENTS REPORT, semaine U40, 6 novembre 1973, page 3, réf. no. 59959U, Derwent Publications Ltd., Londres, GB; & JP - A - 73 31558 (WADA SEIMITSU SHI-KEN CO., LTD.) 29-09-1973**

(73) Titulaire: **SUISSOR S.A., 9, rue Franklin, F-49024 Angers (FR)**

(72) Inventeur: **Heurtaux, Michel, 48a, rue Auguste Vacher, F-41200 Romorantin (FR)**

(74) Mandataire: **Ahner, Francis, CABINET REGIMBEAU 26, avenue Kléber, F-75116 Paris (FR)**

## Description

La présente invention concerne le domaine technique des reconstitutions céramo-métalliques dentares. Elle concerne plus particulièrement la composition du verre céramique utilisé pour réaliser la couche superficielle transparente de reconstitution dentaire telle que des couronnes, incrustations, bridges, etc.

Ce type de reconstitution dentaire est habituellement obtenu à partir d'une succession de couches de céramique venant coiffer une chape métallique, par exemple déposée sur le moignon d'une dent réduite après façonnage à la fraise.

Cette succession de couches céramiques se compose d'une couche basale d'opacification, de deux couches intermédiaires connues sous la dénomination de "dentine" et "d'incisal" et d'une couche superficielle transparente destinée à rappeler l'éclat de l'émail de la dent naturelle.

L'objet de la présente invention vise précisément la réalisation de cette dernière couche superficielle transparente.

Dans l'état actuel de la technique on ne dispose pas de couche céramique dotée d'une parfaite translucidité assimilable à celle d'une vitre. Or, seule une couche superficielle céramique véritablement translucide permet à l'utilisateur de conférer à la prothèse les effets esthétiques de la dent naturelle.

La présente invention a précisément consisté en la mise au point d'une fritte de verre pour réaliser une telle couche céramique rigoureusement transparente.

Conformément à la présente invention, cette fritte est caractérisée en ce quelle est obtenue à partir d'un mélange contenant au moins deux frittes de verre séparées, à savoir:

- environ 90 % en poids d'une première fritte de verre fusible et ne dévitrifiant pas, et

- environ 10 % en poids d'une seconde fritte de verre plus réfractaire et dévitrifiant partiellement en leucite, ledit mélange ayant une granulométrie maximum inférieure à 65 μm et répondant à la composition globale suivante:

| | |
|---|---|
| $SiO_2$ | 59 à 59,5 % en poids |
| $Al_2O_3$ | 15 a 16 % en poids |
| CaO | 0,5 à 1,5 % en poids |
| MgO | 0 à 0,2 % en poids |
| $K_2O$ | 11 à 13 % en poids |
| $Na_2O$ | 7 à 8 % en poids |
| $B_2O_3$ | 2,5 à 3,5 % en poids |
| BaO | 0 à 3 % en poids |
| $CaF_2$ | 0,5 à 3 % en poids |
| $TiO_2$ | 0,2 à 0,5 % en poids |

ledit mélange entrant dans la composition d'une pâte prête à l'emploi contenant une quantité appropriée d'un agent plastifiant, apte à être conditionnée dans un tube ou un distributeur de pâte de type aérosol.

D'autres caractéristiques et avantages de la présente invention apparaîtront à la lecture de la description détaillée faite ci-après notamment en s'appuyant sur un exemple illustratif de réalisation pratique.

La présente invention a également eu pour but la mise au point d'une couche céramique transparente présentant des coefficients de dilatation stables et adaptés à l'ensemble des alliages précieux ou non précieux.

Selon une caractéristique essentielle de la présente invention, la fritte de verre utilisée résulte d'un mélange de deux verres séparés, l'une fusible et ne dévitrifiant pas, l'autre légèrement plus réfractaire et dévitrifiant partiellement en leucite, minéral à forte dilatation thermique.

La première fritte de verre fusible et ne dévitrifiant pas est utilisée telle quelle. En revanche, la seconde fritte de verre est dévitrifiée par exemple pendant environ 12 heures à une température de l'ordre de 900°C. Au cours de ce traitement thermique de dévitrification, il apparaît de façon classique un phénomène de cristallisation in situ.

On observera en outre qu'au cours des cuissons successives, le verre fusible, à la limite de saturation en leucite ne dissout pas la leucite du deuxième verre et maintient donc son taux constant. Il en résulte ainsi une dilatation stable.

La dureté de la couche céramique transparente selon la présente invention est également réduite par augmentation du pourcentage de fondants, en privilégiant $NA_2O$. La couche céramique selon la présente invention est en effet constituée à partir d'un mélange de deux frittes de verre contenant globalelement de 20 à 30 % en poids de fondants, parmi lesquels la soude $NA_2O$ est présente à raison d'environ 7 à environ 8 % en poids.

Pour diminuer la rétraction au cours de la cuisson, la répartition granulométrique du mélange des deux frittes de verre a été ajustée à la suite de l'observation suivante. La porosité d'un ensemble de gros grains (40 à 65 μm) est de l'ordre de 40 %. En introduisant des grains de dimensions moyennes (12 à 40 μm) à raison d'environ 50 % en poids par rapport aux gros grains, la porosité chute à environ 20 %. Enfin, si on introduit des grains de dimensions beaucoup plus fins (inférieurs à 5 μm) la porosité devient encore plus faible. Il en résulte donc un retrait de cuisson moins important. Un tel type de répartition granulométrique permet également d'obtenir une meilleure plasticité de la pâte au moment de son utilisation.

Pour la réalisation d'une fritte de verre selon l'invention destinée à la réalisation d'une couche superficielle transparente, il est ainsi souhaitable de faire appel à un profil granulométrique du type suivant:

| | |
|---|---|
| 40 à 65 μm | 40 à 50 % des grains |
| 12 à 40 μm | 30 à 35 % des grains |
| < 12 μm | 15 à 30 % des grains. |

La présente invention concerne également les frittes de verre nécessaires à la réalisation des couches céramiques superficielles transparentes, qui sont présentées sous la forme d'une pâte prête à l'emploi destinée à faciliter le travail de l'utlisateur tout en permettant de réaliser une économie de produit actif. De telles frittes se présentent sous la forme d'une pâte contenant une quantité appropriée d'un agent plastifiant de manière à permettre leur conditionnement dans un tube ou un distributeur de pâte de type aérosol.

On indiquera ci-après un exemple type de formulation de frittes de verre selon l'invention conditionnées dans un distributeur aérosol:

100 parties en poids de produits actifs,
30 à 40 parties en poids d'agent plastifiant,
100 parties en poids d'un agent propulseur, tel qu'un Fréon ®.

Conformément à un mode de réalisation particulier de ce type de frittes de verre, l'agent plastifiant est de préférence choisi parmi l'éther diéthylique du diéthylèneglycol et l'éther méthylique du propylèneglycol.

## Revendications

1. Fritte de verre nécessaire à la réalisation d'une couche céramique superficielle transparente caractérisée en ce qu'elle est obtenue à partir d'un mélange contenant au moins deux frittes de verre séparées, à savoir:

- environ 90 % en poids d'une première fritte de verre fusible et ne dévitrifiant pas, et
- environ 10 % en poids d'une seconde fritte de verre plus réfractaire et dévitrifiant partiellement en leucite, ledit mélange ayant une granulométrie maximum inférieure à 65 $\mu m$ et répondant à la composition globale suivante:

| | |
|---|---|
| $SiO_2$ | 59 à 59,5 % en poids |
| $Al_2O_3$ | 15 à 16 % en poids |
| CaO | 0,5 à 1,5 % en poids |
| MgO | 0 à 0,2 % en poids |
| $K_2O$ | 11 à 13 % en poids |
| $Na_2O$ | 7 à 8 % en poids |
| $B_2O_3$ | 2,5 à 3,5 % en poids |
| BaO | 0 à 3 % en poids |
| $CaF_2$ | 0,5 à 3 % en poids |
| $TiO_2$ | 0,2 à 0,5 % en poids |

ledit mélange entrant dans la composition d'une pâte prête à l'emploi contenant une quantité appropriée d'un agent plastifiant, apte à être conditionnée dans un tube ou un distributeur de pâte de type aérosol.

2. Fritte selon la revendication 1, caractérisée en ce que ledit mélange des deux frittes de verre présente un profil granulométrique du type suivant:

40 à 65 $\mu m$    40 à 50 % des grains

12 à 40 $\mu m$    30 à 35 % des grains
< 12 $\mu m$    15 à 30 % des grains

3. Fritte de verre selon la revendication 1 ou 2, caractérisée en ce que l'agent plastifiant est choisi parmi l'éther diéthylique du diéthylèneglycol et l'éther méthylique du propylèneglycol.

4. Fritte de verre nécessaires à la réalisation d'une couche céramique selon l'une des revendications 1 à 3, caractérisée en ce qu'elle se présente sous la forme d'une pâte prête à l'emploi contenant une quantité appropriée, apte à être conditionnée dans un tube ou un distributeur de pâte de type aérosol.

5. Fritte de verre selon la revendication 4, caractérisée en ce que l'agent plastifiant est choisi parmi l'éther diéthylique du diéthylèneglycol et l'éther méthylique du propylèneglycol.

## Claims

1. Glass frit required for producing a transparent ceramic surface layer, characterized in that it is obtained from a mixture containing at least two separate glass frits, namely:

- approximately 90 % by weight of a first glass frit which is fusible and does not devitrify, and
- approximately 10 % by weight of a second glass frit which is more refractory and devitrifies partially to leucite, the said mixture having a maximum particle size of less than 65 $\mu m$ and corresponding to the following overall composition:

| | |
|---|---|
| $SiO_2$ | 59 to 59.5 % by weight |
| $Al_2O_3$ | 15 to 16 % by weight |
| CaO | 0.5 to 1.5 % by weight |
| MgO | 0 to 0.2 % by weight |
| $K_2O$ | 11 to 13 % by weight |
| $Na_2O$ | 7 to 8 % by weight |
| $B_2O_3$ | 2.5 to 3.5 % by weight |
| BaO | 0 to 3 % by weight |
| $CaF_2$ | 0.5 to 3 % by weight |
| $TiO_2$ | 0.2 to 0.5 % by weight |

the said mixture forming part of the composition of a ready-for-use paste containing an appropriate quantity of a plasticizing agent and capable of being packaged in a tube or a paste dispenser of the aerosol type.

2. Frit according to claim 1, characterized in that the said mixture of the two glass frits has a particle size distribution of the following type:

40 to 65 $\mu m$    40 to 50 % of the particles
12 to 40 $\mu m$    30 to 35 % of the particles
< 12 $\mu m$    15 to 30 % of the particles.

3. Glass frit according to claim 1 or 2, characterized in that the plasticizing agent is chosen from diethylene glycol diethyl ether and propylene glycol methyl ether.

4. Glass frit required for producing a ceramic layer according to one of claims 1 to 3, characterized in that it is presented in the form of a ready-for-use paste containing an appropriate quantity, capable of being packaged in a tube or a paste dispenser of the aerosol type.

5. Glass frit according to claim 4, characterized in that the plasticizing agent is chosen from diethylene glycol diethyl ether and propylene glycol methyl ether.

## Patentansprüche

1. Glasfritte, die zum Ausbilden einer transparenten keramischen Oberflächenschicht benötigt wird, dadurch gekennzeichnet, daß sie ausgehend von einem Gemisch erhalten wird, das wenigstens zwei getrennte Glasfritten, nämlich etwa 90 Gewichtsprozent einer schmelzbaren und nicht entglasbaren ersten Glasfritte und etwa 10 Gewichtsprozent einer hochschmelzenden und partiell in Leucit entglasbaren zweiten Glasfritte enthält, wobei dieses Gemisch eine maximale Korngröße von weniger als 65 μm und die folgende Gesamtzusamensetzung hat:

| | |
|---|---|
| $SiO_2$ | 59 bis 59,5 Gewichtsprozent |
| $Al_2O_3$ | 15 bis 16 Gewichtsprozent |
| CaO | 0,5 bis 1,5 Gewichtsprozent |
| MgO | 0 bis 0,2 Gewichtsprozent |
| $K_2O$ | 11 bis 13 Gewichtsprozent |
| $Na_2O$ | 7 bis 8 Gewichtsprozent |
| $B_2O_3$ | 2,5 bis 3,5 Gewichsprozent |
| BaO | 0 bis 3 Gewichtsprozent |
| $CaF_2$ | 0,5 bis 3 Gewichtsprozent |
| $TiO_2$ | 0,2 bis 0,5 Gewichtsprozent |

und das besagte Gemisch die Form einer verwendungsfertigen Paste einnimmt, die eine angemessene Menge eines Plastifikationsmittels enthält und in einer Tube oder einem Pastenspender vom Aerosoltyp aufgenommen werden kann.

2. Fritte nach Anspruch 1, dadurch gekennzeichnet, daß das besagte Gemisch der beiden Glasfritten ein Korngrößenprofil folgenden Typs hat:

40 bis 65 μm 40 bis 50 Prozent der Körner
12 bis 40 μm 30 bis 35 Prozent der Körner
< 12 μm 15 bis 30 Prozent der Körner.

3. Glasfritte nach Anspruch 1 oder 2, dadurch gekennzeichnet, daß das Plastifikationsmittel aus Diethyldiethylenglycol oder Methylpropylenglycol gewählt ist.

4. Glasfritte, die zur Ausbildung einer keramischen Schicht benötigt wird, nach einem der Ansprüche 1 bis 3, dadurch gekennzeichnet, daß sie die Form einer verwendungsfertigen Paste einnimmt, die eine angemessene Menge (eines Plastifikationsmittels) enthält und somit in einer Tube oder einem Pastenspender vom Aeorosoltyp aufgenommen werden kann.

5. Glasfritte nach Anspruch 4, dadurch gekennzeichnet, daß das Plastifikationsmittel aus Diethyldiethylenglycol oder Methylpropylenglycol gewählt ist.